# EUROPEAN PATENT SPECIFICATION

(11) **EP 1 238 967 B1**
(45) Date of publication and mention of the grant of the patent: **27.04.2005**
(21) Application number: 01301840.3
(22) Date of filing: 28.02.2001
(51) Int. Cl.: C07C 253/30

(54) **A process for the preparation of 1-(cyano(aryl)methyl) cyclohexanol**
Verfahren zur Herstellung von 1-(Cyan(aryl)methyl)cyclohexanol
Procédé de préparation du 1-(cyano(aryl)méthyl)cyclohexanol

(43) Date of publication of application: 11.09.2002
(73) Proprietor: Council of Scientific and Industrial Research, New Delhi 110 001 (IN)
(72) Inventor: Chavan, Subhash Prataprao, Pune 411 008, Maharashtra (IN); Kamat, Subhash Krishnaji, Pune 411 008, Maharashtra (IN); Balakrishnan, Kamalam, Pune 411 008, Maharashtra (IN); Khobragade, Dushant Anandrao, Pune 411 008, Maharashtra (IN); Thottapillil, Ravindranathan, Pune 411 008, Maharashtra (IN); Gurjar, Mukund Keshao, Pune 411 008, Maharashtra (IN); Kalkote, Uttam Ramrao, Pune 411 008, Maharashtra (IN); Sivadasan, Latha, Pune 411 008, Maharashtra (IN)
(74) Representative: Manaton, Ross Timothy

(56) References cited:
- WO-A-97/20810
- FR-A- 1 382 753
- GB-A- 2 227 743
- DATABASE CHEMABS [Online] CHEMICAL ABSTRACTS SERVICE, COLUMBUS, OHIO, US; Abstract 133:17266, 1999 CHENG, G & ZHUO, C: "Synthesis of 1-[2-amino-1-(p-methoxybenzyl)ethyl]cycloh exanol" XP002168521 & CN 1 225 356 A (EAST CHINA SCIENCE & ENGINEERING UNIVERSITY) 11 August 1999 (1999-08-11)

## Description

### Field of the invention

The present invention relates to a process for the preparation of 1-[(cyano)arylmethyl] cyclohexanol of the general formula 1 from arylacetonitriles of the general formula 3. More particularly the present invention relates to the preparation of 1-[cyano (4-methoxyphenyl) methyl] cyclohexanol of the formula 1b wherein R1 =OMe, R₂ = H which is a key intermediate for the preparation of Venlafaxine of the general formula 2b wherein R1 =OMe and R2 =H and salts thereof which are well known antidepressants of the central nervous system. 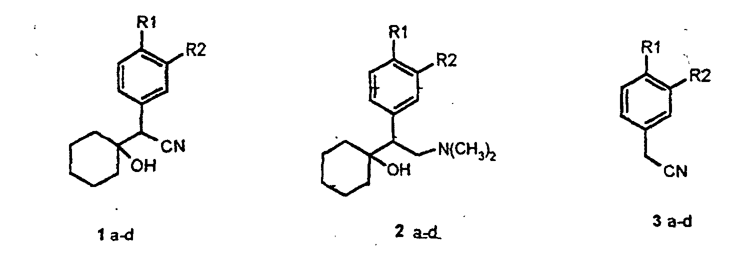
a) R1 = H, R2 =H
b) R1 =OMe R2 =H
c) R1 =OMe R2 =OMe
d) R1 =OMe R2 =cyclopentyloxy

### Background of the invention

In the prior art [Husbands et al. U.S. Patent No.4, 535, 186 (1985) and EP 0112669B] various 2-aryl- (1-hydroxycyclohexyl)-acetonitriles having formula (1) were prepared with <50% yield by the condensation of arylacetonitriles having formula (3) with cyclohexanone using n-butyl lithium as the base at -70°C [Sauvetre et al. Tetrahedron 34 2135 (1978)].

UK Patent No GB 2 227 743 A (1990) of Peter Gerald Shepherd discloses the condensation of compounds having formula 3 with cyclohexanone using lithium diisopropylamide in hydrocarbon solvents like hexane, toluene or cyclohexane at ambient temperature thereby improving the yield to 79%.

The use of butyllithium causes great inconvenience in large-scale preparation since butyllithium is very hazardous. The need for setting up plants for operating at very low temperatures combined with the high cost of butyllithium make this method unacceptable for industrial preparations.

The abstract of CN-A-1,225,356 (published Chemical Abstract 133:17266) also discloses the use of organic bases in the reaction of a phenylacetonitrile (4-methoxyphenylacetonitrile) with cyclohexanone, to produce 1-[2-amino-1-(p-methoxybenzyl)ethyl]cyclohexanol. The reaction additionally requires the use of NaBH₄ and BF₃.

WO 97/20810 discloses a process for the preparation of arylbutylnitrile derivatives which involves reaction of a solution of a 1,3-dihalopropane and a cyanobenzyl derivative in a substantially dimethyl sulphoxide-free solvent with a suspension of a base (such as sodium hydroxide or potassium hydroxide) in a substantially dimethyl sulphoxide-free solvent, at a temperature of at least 35°C. FR 1,382,753 also discloses a method involving the use of dihaloalkanes as alkylating agent and an alkali metal hydroxide as base, at temperatures in excess of 20°C.

### Objects of the invention

The main object of the present invention is to provide a simple and convenient method of preparation of compounds having formula 1(a-d).

It is another object of the invention to provide a process that avoids the use of expensive and hazardous reagents like n-butyl lithium, lithiumdiisopropylamide.

It is still another object of the invention to provide a process that avoids the use of dry solvents like THF and diethyl ether that are expensive and hazardous.

It is yet another objective of the invention to provide a process that does not require elaborate work up or purification processes like chromatography for the isolation of products.

It is a further object of the invention to provide a process by which near quantitative yields of the product are obtained.

It is another object of the invention to provide a process which is simple, easy to handle, inexpensive and non-hazardous so that large-scale production is possible.

### Summary of the invention

Accordingly, the present invention relates to a process for the preparation of 1-(cyano)arylmethyl] cyclohexanol of the general formula 1(1a-d), said process comprising reacting cyclohexanone with the carbanions of an aryl acetonitrile of the general formula 3 (3a-d), 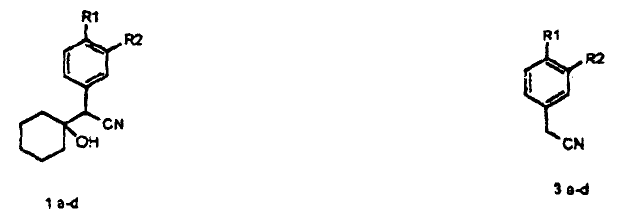
a) R1 = H, R2 =H
b) R1 =OMe R2 =H
c) R1 =OMe R2 =OMe
d) R1 =OMe R2 =cyclopentyloxy
using a base selected from sodium hydroxide and potassium hydroxide at temperature in the range of 0-15°C for a time period in the range of 15 minutes to 120 minutes, isolating the compound of formula 1 and purifying the compound of formula 1(1a-d) by crystallisation.

In one embodiment of the invention the base used is selected form the group consisting of powdered sodium hydroxide, powdered potassium hydroxide, 10% aqueous sodium hydroxide solution, 10% aqueous potassium hydroxide solution and 50% sodium hydroxide solution.

In another embodiment of the invention the quantity of base used is in the range of 0.25 mole to 1 mole.

In a further embodiment of the invention the quantity of base used is 0.5 mole.

In another embodiment of the invention, the reaction is carried out in the presence of or absence of a phase transfer catalyst.

In another embodiment of the present invention, the phase transfer catalyst used is selected from the group consisting of tetrabutylammonium hydrogensulphate, tetrabutylammonium bromide, tetrabutylammonium chloride, and tetrabutylammonium iodide or benzyltriethyl ammonium chloride.

In still another embodiment of the present invention, the aryl acetonitrile of formula 3 used is selected from the group consisting of phenylacetonitrile, 4-methoxyphenylacetonitrile, 3,4-dimethoxy phenylacetonitrile or 3-cyclopentyloxy, 4-methoxy phenylacetonitrile.

### Detailed description of the invention

The process of the present invention is described by the following examples, which are illustrative only and should not be construed as limit to the scope of the reaction in any manner.

### Example 1

Phenylacetonitrile 3a (11.7 parts, 0.1 moles) was placed in a beaker and cooled to 0°C. Powdered potassium hydroxide (0.28 parts, 0.05 moles catalytic quantity) and tetrabutylammonium iodide (0.37 parts, 0.01 moles) was added and mixed thoroughly. After 5 minutes, cyclohexanone (9.8 parts, 0.1 moles) was added slowly at 0°C. The mixture on stirring vigorously for 15 minutes solidified suddenly. Water (100 parts) was added at this stage and the reaction mixture stirred for an hour to complete the reaction. The product- 1-cyano [(aryl) methyl] cyclohexanol 1a was filtered by suction, washed with water (3x100 parts) and dried to constant weight. 18.7 parts, (87%). Crystallized from ethyl acetate pet ether to yield shining white crystals Mp.101-2 °C. NMR (200 MHz., CDCl₃) δ 7.4(s, 5H, aromatic); 3.80 (s, 1H CH CN); 1.59 (m, 10H cyclohexyl); 1.24 (br s, 1H). M+215

### Example 2

4-Methoxyphenylacetonitrile 3b (147 parts, 1 mole) was cooled to 0°C, and stirred vigorously with a solution of 10% aqueous sodium hydroxide solution (100 parts, 0.25 mol). Tetrabutyl ammonium hydrogen sulphate (4.0 parts, 0.1 mol) was added in one lot while stirring. The reaction was warmed to 15°C after which, cyclohexanone (100 parts 1.02 mol) was added rapidly in 10 minutes taking care to keep the temperature of the reaction below 15°C. A thick smooth solid separates. At this stage the solid was crushed to fine pieces and water (1000 parts) was added to facilitate stirring. Stirring was continued for 2 hrs. When the reaction was over, (monitored by tlc) the solid was filtered, washed free of alkali and dried to constant weight. Yield 162 parts (97.6%) .The product 1-cyano [(4-methoxyphenyl) methyl]-cyclohexanol 1b was crystallized from ethylacetate pet.ether to obtain shining white needles of >99% purity on HPLC M.p. 125-6°C. (litt. mp 123-5°). ¹HNMR (CDCl₃) δ 7.32, 6.95 (4H, q, p-substituted aromatic), 3.8 (3H, s - OCH₃); 3.76 (1H, s, CH CN 1.56 (10 H, m, aliphatic cyclohexyl); M⁺245

### Example 3

3,4-Dimethoxyphenylactonitrile 3c (0.885 parts, 0.005 moles) was cooled to 15°C in a beaker and stirred vigorously with powdered sodium hydroxide (0.100 parts 0.25 mol). Trimethylbenzylammonium chloride (0.028 parts, 0.003 moles) was added to this mixture followed by cyclohexanone (0.5 parts, 0.005 moles). Stirring was continued for 2 hrs and the reaction was left overnight at 0°C. Water (5ml) was added after which the solid that separated was filtered, washed with water and dried 0.976 parts (71%). The product 1-cyano [(3,4-dimrthoxyphenyl) methyl] acetonitrile 1c was crystallised from ethylacetate to fine needles. Mp.134-5° C. NMR: (CDCl₃) δ 6.85 (m, 3H, aromatic). 3.87(s, 3H O CH₃); 3.86(s, 3H O CH₃); 3.69(s, 1H, CH CN); 1.59 (m, 10H, cyclohexyl); 1.24 (bs, 1H, OH); M⁺275

### Example 4

3-cyclopentyloxy, 4-methoxy phenylacetonitrile 3d (0.3 parts, 0.00129 mols) was cooled to 0°C and stirred with a 50% solution of sodium hydroxide (0.1 part, 0.00125 moles) and tetrabutylammoniumhydrogensulphate (0.044 parts, 0.000129 mol). Cyclohexanone (0.129 parts 0.0013 moles) was added after 10 minutes and stirring continued for 2 hours to ensure completion of the reaction. The product 1-cyano- [(3-cyclopentyloxy, 4methoxyphenyl) methyl] cyclohexanol 1d was isolated by filtration, washing and drying as a low melting solid. NMR (CDCl₃) δ (6.8 (m, 3H, aromatic); 4.75 (br. S, 1H, O-CH-); 3.6 (s 3H, OMe); 3.59 (s, 1H CH-CN); 1.5-2 (m, 18H, cyclopentyl and cyclohexyl). M⁻ 329

### Example 5

4-Methoxyphenylacetonitrile 1b (1.47parts, 0.1mol) was cooled to 0°C and stirred with powdered sodium hydroxide (0.02 parts 0.05 mol). Tetrabutylammoniumiodide (0.036 parts 0.01 mol) was added to this mixture and condensed with cyclohexanone (0.98 parts, 0.1 mol) as described above. The product 1b was isolated by filtration, washing and drying of the solid obtained after stirring the reaction mixture for 10 minutes. (2.2 parts, 89.7%)

### Example 6

4-Methoxyphenylacetonitrile 1b (1.47parts, 0.1mol) was cooled to 0°C and stirred with powdered sodium hydroxide (0.100 parts.0.025 mol) without any phase transfer catalyst. When the reaction became thick, cyclohexanone (0.98 parts, 0.1 mol) was added and the reaction stirred for another 10 minutes till solid separated. The product Ib was isolated as described above. (1.91 part, 78%)

### The advantages of the present invention are as follows:

The present invention avoids the use of expensive and hazardous reagents like n-butyl lithium, lithiumdiisopropylamide.

The present invention also avoids the use of dry solvents like THF and diethyl ether, which are also expensive and hazardous. The present method can be conducted in water or even under solvent free conditions.

The present invention does not involve elaborate work up or purification processes like chromatography for the isolation of products.

The present invention avoids the inconvenience of carrying out reactions at very low temperatures.

The present invention does not require the use of inert atmosphere.

The present invention describes a process by which near quantitative yields of the product are obtained.

The present invention describes a process, which is simple, easy to handle, and non-hazardous so that large-scale production is possible.

The present invention presents a very convenient method of preparation of 1 through a simple short and cost effective process.

## Claims

1. A process for the preparation of 1-[(cyano) aryl methyl] cyclohexanol of the general formula 1(a-d), by reacting cyclohexanone with the carbanions of an aryl acetonitrile of the general formula 3(a-d) 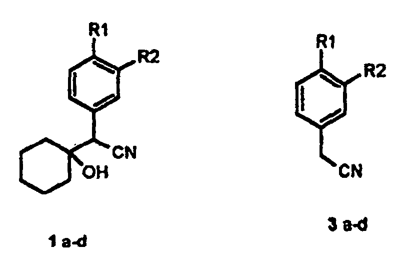
a) R1 = H, R2 =H
b) R1 =OMe R2 =H
c) R1 =OMe R2 =OMe
d) R1 =OMe R2 =cyclopentyloxy
using a base selected from sodium hydroxide and potassium hydroxide at a temperature in the range of 0 to 15°C for a time period in the range of 15 minutes to 120 minutes, isolating the compound of formula 1 and purifying the compound of formula 1(a-d) by crystallisation.

2. A process as claimed in claim 1, wherein the base used is selected from the group consisting of powdered sodium hydroxide, powdered potassium hydroxide, 10% aqueous sodium hydroxide solution, 10% aqueous potassium hydroxide solution and 50% aqueous sodium hydroxide solution.

3. A process as claimed in claim 1 or claim 2, wherein the base is used in an amount in the range of 0.25 mole to 1 mole.

4. A process as claimed in claim 3, wherein the quantity of base used is 0.5 mole.

5. A process as claimed in any preceding claim, wherein the reaction is carried out in the presence of a phase transfer catalyst.

6. A process as claimed in claim 5 wherein the phase transfer catalyst used is selected from the group consisting of tetrabutylammonium hydrogensulphate, tetrabutylammonium bromide, tetrabutylammonium chloride, tetrabutylammonium iodide and benzyltriethyl ammonium chloride.

7. A process as claimed in any preceding claim, wherein the aryl acetonitrile of formula 3 is selected from the group consisting of phenylacetonitrile, 4-methoxyphenylacetonitrile, 3,4-dimethyloxyphenylacetonitrile and 3-cyclopentyloxy, 4-methoxy phenylacetonitrile.

8. A process as claimed in any of claims 1 to 4, wherein the process is carried out in the absence of a phase transfer catalyst.

9. A process as claimed in any preceding claim, wherein the solvent used is water.

10. A process as claimed in claim 1, wherein the base is used in a catalytic amount.

## Patentansprüche

1. Verfahren zur Herstellung von 1-[(Cyano)arylmethyl]cylohexanol der allgemeinen Formel 1(a-d), bei dem man Cyclohexanon mit den Carbanionen eines Arylacetonitrils der allgemeinen Formel 3(a-d) 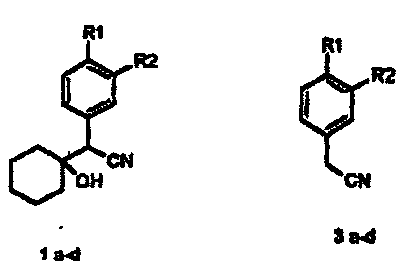
a) R1 = H, R2 = H
b) R1 = OMe, R2 = H
c) R1 = OMe, R2 = OMe
d) R1 = OMe, R2 = Cyclopentyloxy
unter Verwendung einer unter Natriumhydroxid und Kaliumhydroxid ausgewählten Base bei einer Temperatur im Bereich von 0 bis 15°C über einen Zeitraum im Bereich von 15 Minuten bis 120 Minuten umsetzt, die Verbindung der Formel 1 isoliert und die Verbindung der Formel 1(a-d) durch Kristallisation reinigt.

2. Verfahren nach Anspruch 1, bei dem man die verwendete Base aus der Gruppe bestehend aus Natriumhydroxidpulver, Kaliumhydroxidpulver, 10%iger wäßriger Natriumhydroxidlösung, 10%iger wäßriger Kaliumhydroxidlösung und 50%iger wäßriger Natriumhydroxidlösung auswählt.

3. Verfahren nach Anspruch 1 oder 2, bei dem man die Base in einer Menge von 0,25 mol bis 1 mol verwendet.

4. Verfahren nach Anspruch 3, bei dem die verwendete Basenmenge 0,5 mol beträgt.

5. Verfahren nach einem der vorhergehenden Ansprüche, bei dem man die Umsetzung in Gegenwart eines Phasentransferkatalysators durchführt.

6. Verfahren nach Anspruch 5, bei dem man den verwendeten Phasentransferkatalysator aus der Gruppe bestehend aus Tetrabutylammoniumhydrogensulfat, Tetrabutylammoniumbromid, Tetrabutylammoniumchlorid, Tetrabutylammoniumiodid und Benzyltriethylammoniumchlorid auswählt.

7. Verfahren nach einem der vorhergehenden Ansprüche, bei dem man das Arylacetonitril der Formel 3 aus der Gruppe bestehend aus Phenylacetonitril, 4-Methoxyphenylacetonitril, 3,4-Dimethyloxyphenylacetonitril und 3-Cyclopentyloxy-4-methoxyphenylacetonitril auswählt.

8. Verfahren nach einem der Ansprüche 1 bis 4, das man ohne Phasentransferkatalysator durchführt.

9. Verfahren nach einem der vorhergehenden Ansprüche, bei dem man als Lösungsmittel Wasser verwendet.

10. Verfahren nach Anspruch 1, bei dem man die Base in einer katalytisch wirksamen Menge verwendet.

## Revendications

1. Procédé de préparation de 1-[(cyano)arylméthyl]cyclohexanol de formule générale 1(a-d), par réaction de la cyclohexanone avec les carbanions d'un arylacétonitrile de formule générale 3(a-d) 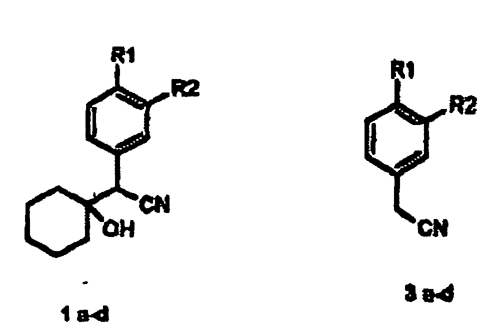
a) R1 = H, R2 = H
b) R1 = OMe, R2 = H
c) R1 = OMe, R2 = OMe
d) R1 = OMe, R2 = cyclopentyloxy
en utilisant une base choisie parmi l'hydroxyde de sodium et l'hydroxyde de potassium à une température dans la plage de 0 à 15 °C pendant une période de temps dans la plage de 15 minutes à 120 minutes, isolation du composé de formule 1 et purification du composé de formule 1(a-d) par cristallisation.

2. Procédé selon la revendication 1, dans lequel la base utilisée est choisie parmi le groupe constitué de l'hydroxyde de sodium pulvérulent, de l'hydroxyde de potassium pulvérulent, d'une solution aqueuse d'hydroxyde de sodium à 10 %, d'une solution aqueuse d'hydroxyde de potassium à 10 % et d'une solution aqueuse d'hydroxyde de sodium à 50 %.

3. Procédé selon la revendication 1 ou la revendication 2, dans lequel la base est utilisée en une quantité dans la plage de 0,25 mol à 1 mol.

4. Procédé selon la revendication 3, dans lequel la quantité de base utilisée est de 0,5 mol.

5. Procédé selon l'une quelconque des revendications précédentes, dans lequel la réaction est effectuée en présence d'un catalyseur à transfert de phase.

6. Procédé selon la revendication 5, dans lequel le catalyseur à transfert de phase utilisé est choisi parmi le groupe constitué de l'hydrogénosulfate de tétrabutylammonium, du bromure de tétrabutylammonium, du chlorure de tétrabutylammonium, de l'iodure de tétrabutylammonium et du chlorure de benzyltriéthylammonium.

7. Procédé selon l'une quelconque des revendications précédentes, dans lequel l'arylacétonitrile de formule 3 est choisi parmi le groupe constitué du phénylacétonitrile, du 4-méthoxyphénylacétonitrile, du 3,4-diméthyloxyphénylacétonitrile et du 3-cyclopentyloxy-4-méthoxyphénylacétonitrile.

8. Procédé selon l'une quelconque des revendications 1 à 4, dans lequel le procédé est réalisé en l'absence d'un catalyseur de transfert de phase.

9. Procédé selon l'une quelconque des revendications précédentes, dans lequel le solvant utilisé est l'eau.

10. Procédé selon la revendication 1, dans lequel la base est utilisée en une quantité catalytique.
